Europäisches Patentamt

European Patent Office

Office européen des brevets

(11) Publication number: **0 134 847**
**B1**

# (12) EUROPEAN PATENT SPECIFICATION

(45) Date of publication of patent specification: **27.05.87**

(21) Application number: **83304452.2**

(22) Date of filing: **02.08.83**

(51) Int. Cl.⁴: **A 61 M 15/00**

(54) Inhalation valve.

(43) Date of publication of application:
**27.03.85 Bulletin 85/13**

(45) Publication of the grant of the patent:
**27.05.87 Bulletin 87/22**

(84) Designated Contracting States:
**AT BE CH DE FR GB IT LI LU NL SE**

(56) References cited:
**EP-A-0 009 667**
**DE-A-1 902 917**
**GB-A-2 110 543**
**US-A-3 556 122**

(73) Proprietor: **Trutek Research Inc.**
**350 Telser Road**
**Lake Zurich Illinois 60047 (US)**

(72) Inventor: **Nowacki, Christopher**
**4027 N. Terramere**
**Arlington Heights Illinois (US)**
Inventor: **Brisson, Alfred G.**
**22358 Timberlea**
**Kildeer Illinois 60047 (US)**

(74) Representative: **Carpenter, David et al**
**MARKS & CLERK Alpha Tower Suffolk Street**
**Queensway**
**Birmingham B1 1TT (GB)**

Courier Press, Leamington Spa, England.

## Description

A person suffering from asthma may when suffering an asthmatic attack have rather considerable trouble in breathing, due to swelling in the bronchi and due to secretion of mucus. There are various antiasthmatic pills that are effective, but which generally are somewhat slow-acting. There are also medications available for intravenous treatment which work quite rapidly, but which require administration by skilled medical personnel. For most patients the promptest, immediately available relief is by way of an inhalant. Epinephrine or other suitable asthmatic medication is packaged with a suitable diluent in a small pressurized cannister or cartridge which interfits with a mouthpiece. The patient places the mouthpiece in his mouth, and depresses the cartridge, thereby releasing a measured amount of medication which is inhaled through the mouthpiece.

Some patients do not inhale properly, and the mouthpiece may not be completely effective in cooperation with the cartridge to convert the medication into a mist which is deposited in the proper bronchial area to relieve the asthmatic attack. Often there are small droplets, rather than a mist, and this may be compounded by improper inhalation which results in much of the medication simply going into the throat and stomach where it is ineffective against the asthmatic attack.

United States of America Patent No. 3556122 illustrates an inhalation valve for use with an artificial respiration apparatus. There is no suggestion in US—A—3556122 that the valve would be suitable for use in a medicament inhaler. Moreover the valve of US—A—3556122 utilizes a shaped flexible valve member which is complex and expensive to produce and which, when used as taught in US—A—3556122 is not totally reliable in high pressure exhalation conditions.

Objects and summary of the invention

Thus, an principal object of the present invention is to provide an improved valve and mouthpiece structure for converting epinephrine or other broncho dilator into a proper mist for inhalation by a person suffering an asthmatic attack, and wherein the difficulties and disadvantages of the value disclosed in US patent 3556122 are obviated.

It is a further object to the present invention to provide a valve as just noted which is cooperable with a large number of existing commercial broncho-dilator mouthpieces for providing improved mist and inhaling by the patient.

It further is an object of the present invention to provide a universal extension fitting for bronchodilators including a one-way valve which opens upon inhalation without bypassing any ambient air into the inhaled mist, and which closes upon exhalation with the exhaled breath bypassed and not entering into the chamber containing the broncho-dilator mist.

In accordance with the present invention there is provided an inhalation valve including an elongate body defining a passageway having an entering upstream end and an exit downstream end, means at said entering end for receiving structure for applying inhalation medication, a mouthpiece at said exit end for receipt in a patient's mouth, an elastomeric diaphragm having a diametral opening therein and an imperforate portion outwardly of the diametral opening, means engaging and gripping said diaphragm adjacent the periphery thereof and mounting said diaphragm adjacent said exit end transversely of said passageway, said diaphragm flexing downstream and freeing said opening for passage of medication upon inhalation by a patient, backup means immediately upstream of said diaphragm, a cylindrical flange extending upstream from said mouthpiece and terminating at a rim presenting a valve seat normally spaced from said diaphragm downstream thereof, exhaled air from said mouthpiece passing between said rim and said diaphragm, and exhaust port means disposed outwardly of said cylindrical flange to exhaust exhaled air to the atmosphere, said backup means supporting said diaphragm during exhalation by a patient to prevent flexing of said diaphragm upstream and including an elongate member aligned with said diametral opening of said diaphragm and against which said diaphragm is pressed during exhalation to seal said opening to prevent retrograde flow upon exhalation, and in that said diaphragm is substantially planar, whereby upon inhalation the imperforate portion of the diaphragm which is presented to the rim of the valve seat being moved into engagement with said valve seat rim, to seal off said exhaust port means, and upon exhalation said imperforate portion of said diaphragm disengages from the valve seat rim, to expose said exhaust port means.

Thus in a preferred embodiment there is provided an extension fitting for a broncho-dilator having an elastomeric receiver fitting about the existing mouthpiece of a commercial broncho-dilator device. The receiver is at the entering end of a cylinder having a mouthpiece at its opposite end. An elastomeric valve is provided between the cylinder and the mouthpiece, the valve comprising a generally flat diaphragm having a slit therein. Upon inhalation the diaphragm flexes to permit opening of the slit so that mist will pass through the cylinder into the bronchial passages of the asthmatic sufferer. When the patient exhales the diaphragm is forced flat against a supporting spider with the slit effectively sealed. Bypass openings are provided to permit exhaust of the exhaled breath into the atmosphere. These exhaust openings are closed by the diaphragm upon inhalation so that only the epinephrine or the like mist is inhaled without being mixed with ambient air.

The drawings

The invention will best be understood with reference to the following text when taken in connection with the accompanying drawings, wherein:

Fig. 1 is a view illustrating a broncho dilator device as found in the prior art.

Fig. 2 is a side view partly in longitudinal section illustrating an inhalation valve in accordance with one example of the present invention.

Fig. 3 is a cross-sectional view taken substantially along the line 3—3 in Fig. 2 illustrating the spider which backs up the elastomeric valve diaphragm; and

Fig. 4 is a cross-section view taken substantially along the line 4—4 showing the elastomeric diaphragm.

Detailed disclosure of the illustrative embodiment

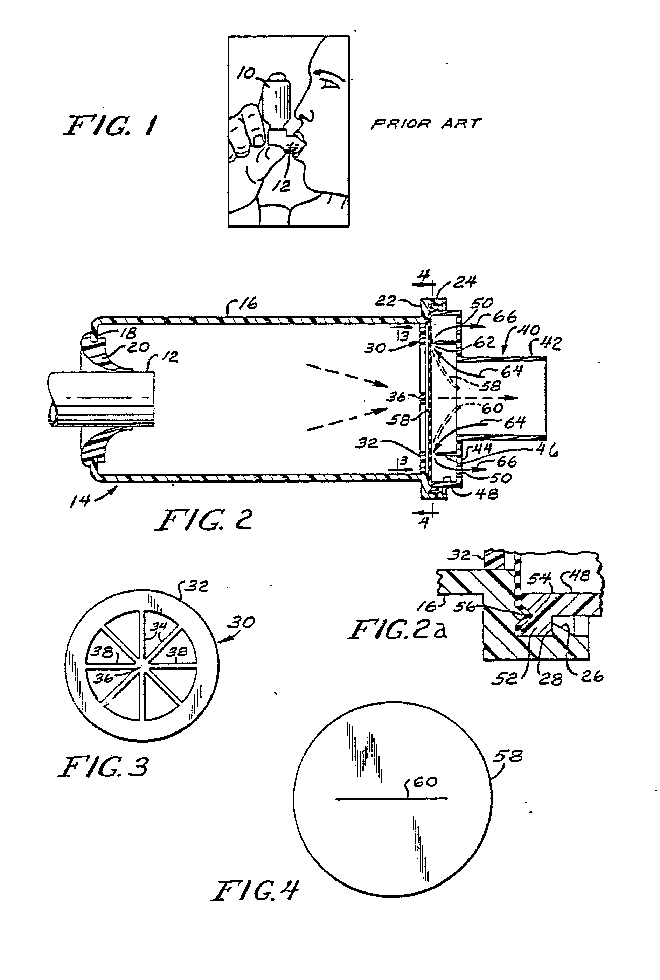

Reference first should be made to Fig. 1 for an understanding of the prior art. A small pressurized cannister or cartridge, sometimes referred to as a nebulizer, is charged with epinephrine or other suitable antiasthmatic medication in a suitable diluent, and under pressure. The cartridge fits into a receiving end of a right angle mouthpiece 12, the opposite end of which is placed in the asthmatic sufferer's mouth. The cartridge is pressed down, being squeezed between the index finger and the thumb underlying the mouthpiece. This causes a valve stem in the cartridge to press against a reaction base in the mouthpiece to discharge a measured quantity of medication into the mouthpiece. The discharge is supposed to be in a form of a mist, but in fact often contains small droplets. The patient inhales, and the mist passes into the mouth, and hopefully into the bronchial tubes to provide asthmatic relief. The patient is then supposed to hold his breath for a short time, and subsequently to inhale slowly through nearly closed lips. However, as noted heretofore, some of the medication may simply be in the form of droplets rather than mist, and the droplets generally do not reach the bronchial tubes to effect their intended purpose.

We have found that the drops can be broken up into a mist, and the patient can be more or less forced to inhale properly with the use of the inhalation valve forming the subject matter of the present invention, and for which reference should be had particularly to Fig. 2, and also to Figs. 3 and 4.

As shown in Fig. 2 there is an inhalation valve 14 comprising a cylinder 16 preferably molded of a suitable plastic material. The cylinder is provided at its entering end (the left end in Fig. 2) with a radially inwardly directed flange 18 of limited extent. This flange retains a generally frustoconical elastomeric adapter 20 which receives the mouthpiece 12 previously referred to. The frustoconical shape and the elastomeric nature of the adapter 20 is such that mouthpieces of widely differing sizes and configurations can be securely gripped.

At the opposite end of the cylinder 16 there is an outwardly extending peripheral flange 22 having at its extremity an axially extending cylindrical flange 24. At its extremity the cylindrical flange 24 is provided with an internal taper 26 having a right angle shoulder or stop surface 28 behind it; 24, 26 can be spaced teeth.

Also at the exit end of the cylinder there is provided a spider 30 which is shown also in Fig. 3. The spider 30 may be molded integrally with the cylinder 16, but more conveniently is a separate plastic piece which is secured within the cylinder by known techniques, such as a cement, sonic welding, etc. The spider comprises an annular ring 32 having formed integrally therewith a plurality of radial ribs 34 joined together at the center at 36. As shown in Fig. 3 there are eight such ribs. The precise number is not critical, but there should be one pair of ribs extending diametrically across the spider, such pair in the present instance being identified by numeral 38. The fitting 14 further includes a mouthpiece element 40 having a generally cylindrical, slightly tapered mouthpipe 42 to be received in the person's mouth. The mouthpipe has at its entering end a radially extending flange or disc 44 which partway out is provided with a cylindrical flange 46 extending in the entering direction, opposite to the mouthpipe 42. At its periphery the disc 44 is provided with a cylindrical flange 48 also extending in the direction of the receiving end of the cylinder 16, i.e., opposite to the mouthpipe 42. Intermediate the cylindrical flanges 46 and 48 the disc 44 is provided with an annular array of spaced apertures 50 for exhaust of exhaled air.

The flange 48 is axially somewhat longer than the flange 46, as will be explained shortly, and it is provided at its extremity with a radially extending annular flange 52 which snaps over the tapered surface 26 and behind the shoulder 28 so that the mouthpiece element 40 is held in assembled position with the cylinder 16. The entering end face of the flange 52 is provided with an annular recess 54, and the confronting face of the flange 22 on the cylinder 16 is provided with a complementary rib 56.

An elastomeric diaphragm 58 is trapped between the flange 52 and the flange 22, being securely held in place by the complementary rib 56 and recess 54. As is particularly seen in Fig. 4, the diaphragm 58 is provided with a diametral slit 60 which in relaxed position lies across the aligned ribs 38. As now will be seen, the relative shortness of the ring or flange 46 as compared with the flange 48 provides for clearance space 62 between the flange 46 and the diaphragm 58.

In order to use the present inhalation valve, the mouthpiece 12 of the prior art is inserted in the member 20, as previously described. The mouthpipe 22 is received in the mouth of the person suffering an asthmatic attack. The cartridge is pressed down in the mouthpiece to release a measured amount of medication, in accordance with the prior art. However, rather than the misted medication passing directly through the mouthpiece into the mouth of the person using it, the mist is passed into the cylinder 16. The elongated flow path provided by the cylinder allows further opportunity for droplets to atomize or evaporate into a mist. As the person inhales, the diaphragm 58 deflects toward the person's mouth, as illustrated in broken lines in Fig. 2, thus allowing the slit 60 to open, and thus to pass the misted medication.

Movement of the medication past the spider 30, and past the edges of the slit, enhances evaporation or dispersion of droplets into the desired mist form. It is not necessary for the patient to engage in any artificial or learned process of breathing, since he need only inhale, and exhale. Upon exhalation the diaphragm returns to its initial flat position with the slit 60 sealing across the aligned ribs 38. Air passes through the clearance space 62 as indicated by the arrows 64, and out through the holes 50 as as indicated by the arrows 66. Upon inhalation the deflection of the diaphragm causes the diaphragm to press against the free end of the cylindrical flange 46, and thereby to prevent any outside air from being drawn in along with the medication. Floating of the diaphragm back and forth upon alternate inhalation and exhalation also aids in breaking up any droplets into mist form.

As noted, the parts are molded of suitable plastic material, with the exception of the diaphragm, which is an elastomeric material; silicone rubber is one particular material that has been found satisfactory for this purpose. The device is readily washed or cleansed in alcohol, and hence is capable of extended use. However, the cost of production is low, and it is not necessary to preserve the device indefinitely.

It will now be apparent that we have invented an improved valve or extension piece for use with a conventional broncho dilator inhalation device which would cooperate with a large number of prior art devices, which is remarkably easy for the asthmatic sufferer to use, and which provides improved formation of mist. The simplicity of inhalation for the asthmatic sufferer, and the improved misting result in more of the antiasthmatic medication ending up in the bronchi for relief of the asthmatic attack, thereby minimizing bypassing of the medication through the throat and stomach where it does no good.

The specific example of the invention as herein shown and described is for illustrative purposes only. Various changes will no doubt occur to those skilled in the art, and will be understood as forming a part of the present invention insofar as they fall within the scope of the appended claims.

## Claims

1. An inhalation valve including an elongated body defining a passageway (16) having an entering upstream end (18) and an exit downstream end (22), means (20) at said entering end for receiving structure (12) for applying inhalation medication, a mouthpiece (40) at said exit end for receipt in a patient's mouth, an elastomeric diaphragm (58) having a diametral opening (60) therein and an imperforate portion outwardly of the diametral opening, means (52) engaging and gripping said diaphragm adjacent the periphery thereof and mounting said diaphragm adjacent said exit end transversely of said passageway, said diaphragm flexing downstream and freeing said opening for passage of medication upon inhalation by a patient, backup means (30) immediately upstream of said diaphragm, a cylindrical flange (46) extending upstream from said mouthpiece and terminating at a rim presenting a valve seat normally spaced from said diaphragm downstream thereof, exhaled air (64) from said mouthpiece passing between said rim and said diaphragm, and exhaust port means (50) disposed outwardly of said cylindrical flange (46) to exhaust exhaled air to the atmosphere, characterized in that said back up means (30) supports said diaphragm during exhalation by a patient to prevent flexing of said diaphragm (58) upstream and includes an elongate member (38) aligned with said diametral opening (60) of said diaphragm and against which said diaphragm is pressed during exhalation to seal said opening (60) to prevent retrograde flow upon exhalation, and in that said diaphragm is substantially planar, whereby upon inhalation the imperforate portion of the diaphragm which is presented to the rim of the valve seat is moved into engagement with said valve seat rim, to seal off said exhaust port means (50), and upon exhalation said imperforate portion of said diaphragm disengages from the valve seat rim, to expose said exhaust port means.

2. An inhalation valve as claimed in claim 1 characterized in that said diametral opening comprises a slit (60).

3. An inhalation valve as claimed in claim 1 or claim 2 characterized in that said mouthpiece includes an annular transverse plate (44), a tubular portion (42) extending downstream from said annular transverse plate adjacent the inner margin thereof for receipt in a patient's mouth, said annular flange (46) being spaced outwardly of said tubular portion and extending upstream from said annular transverse plate.

4. An inhalation valve as claimed in claim 3 characterized in that said exhaust port means comprises openings (50) in said annular transverse plate (44) outwardly of said annular flange (46).

5. An inhalation valve as claimed in anyone of claims 1 to 4 characterized in that said backup means comprises a spider (30) including said elongate member (38).

6. An inhalation valve as claimed in claim 1 characterized in that said receiving means at said entering end comprises an open ended frustoconical elastomeric member (20) secured to the entering end of and extending into said body.

## Patentansprüche

1. Beatmungsventil mit einem länglichen Körper, der einen Durchgang (16) mit einem stromaufwärts liegenden Einlaßende (18) und einem stromabwärts liegenden Auslaßende (22) definiert, vorrichtungen (20) am genannten Einlaßende zur Aufnahme einer Anordnung (12) zum Einführen von Inhalationsmedikation, einem Mundstück (40) am genannten Auslaßende zur Aufnahme im Mund eines Patienten, einer elastomeren Membran (58) mit einer diametralen Öffnung (60) darin und einem nicht durchlochten Bereich außerhalb der diametralen Öffnung, Vorrichtungen (52), die die Membran nahe ihres Umfanges im Eingriff erfassen und die Membran nahe dem Auslaßende quer zum Durchgang halten, wobei sich die Membran stromabwärts dehnen kann und die Öffnung für den Durchtritt von Medikation beim Einatmen eines Patienten freigibt, einer Stützvorrichtung (30) unmittelbar stromaufwärts von der genannten Membran, einem Ringflansch (46), der sich stromaufwärts von Mundstück erstreckt und an einem Rand endet, der einen Ventilsitz bildet, welcher im Normalfall beabstandet stromabwärts von der Membran liegt, wobei ausgeatmete Luft (64) vom Mundstück zwischen dem besagten Rand und der besagten Membran durchtritt, und Auslaßöffnungsvorrichtungen (50) die auswärts von dem besagten Ringflansch (46) angeordnet sind, um ausgeatmete Luft in die Atmosphäre austreten zu lassen, dadurch gekennzeichnet, daß die besagte Stützvorrichtung (30) die genannte Membran beim Ausatmen eines Patienten abstützt, um eine Dehnung der Membran stromaufwärts zu verhindern, und ein längliches Teil (38) umfaßt, welches mit der diametralen Öffnung (60) der genannten Membran fluchtet und gegen welches die Membran beim Ausatmen zum dichten Verschließen der Öffnung (60) gepreßt wird, um einen Rückfluß beim Ausatmen zu verhindern und darin, daß die besagte Membran im wesentlichen eben ist, wodurch beim Einatmen der nicht durchlochte Bereich der Membran, der dem Ventilsitzrand zugewandt ist, in Berührung mit dem Ventilsitz gebracht wird, um die genannten Auslaßöffnungsvorrichtungen (50) zu verschließen, und wodurch beim Ausatmen der nicht durchlochte Bereich der besagten Membran sich vom Ventilsitz abhebt, um die genannten Auslaßöffnungsvorrichtungen freizulegen.

2. Beatmungsventil nach Anspruch 1, dadurch gekennzeichnet, daß die genannte diametrale Öffnung einen Schlitz (60) umfaßt.

3. Beatmungsventil nach Anspruch 1 oder 2, dadurch gekennzeichnet, daß das genannte Mundstück eine ringförmige Querplatte (44) und einen rohrförmigen Bereich (42) umfaßt, welcher sich stromabwärts von der genannten ringförmigen Querplatte nahe deren Innenkante erstreckt, um im Mund eines Patienten aufgenommen zu werden, wobei der Ringflansch (46) auswärts von dem genannten rohrförmigen Bereich beabstandet ist und sich stromaufwärts von der genannten ringförmigen Querplatte erstreckt.

4. Beatmungsventil nach Anspruch 3, dadurch gekennzeichnet, daß die Auslaßöffnungsvorrichtungen Öffnungen (50) in der ringförmigen Querplatte (44) auswärts vom Ringflansch (46) umfassen.

5. Beatmungsventil nach einem der Ansprüche 1 bis 4, dadurch gekennzeichnet, daß die Stützvorrichtung eine Stützscheibe (30) umfaßt, welche das längliche Teil (38) einschließt.

6. Beatmungsventil nach Anspruch 1, dadurch gekennzeichnet, daß die Aufnahmevorrichtungen am Einlaßende ein kegelstumpfförmiges elastomeres Teil (20) mit offenem Ende umfassen, welches am Einlaßende festgelegt ist und sich in den Körper hineinerstreckt.

## Revendications

1. Clapet d'inhalation comprenant un corps allongé qui délimite un passage (16) présentant une extrémité amont d'entrée (18) et une extrémité aval de sortie (22), des moyens (20) disposés à l'extrémité d'entrée pour recevoir une structure (12) permettant d'appliquer une médication d'inhalation, un embout buccal (40) disposé à l'extrémité de sortie et destiné à se loger dans la bouche d'un malade, une membrane élastomère (58) dans laquelle est ménagée une ouverture diamétrale (60) et qui comporte à l'extérieur de cette ouverture diamétrale une partie non perforée, des moyens (52) coopérant, en la serrant, avec la membrane au voisinage de sa périphérie et servant à monter cette membrane à proximité de l'extrémité de sortie et transversalement au dit passage, cette membrane fléchissant vers l'aval et libérant l'ouverture pour le passage de la médication lors d'une inspiration de la part d'un malade, des moyens de retenue (30) situés immédiatement en amont de la membrane, une collerette cylindrique (46) s'étendant en amont de l'embout buccal et se terminant par un rebord qui présente un siège de clapet normalement situé à distance de la membrane en aval de celle-ci, l'air (64) expiré par l'embout buccal passant entre ce rebord et cette membrane, et des moyens formant orifice d'évacuation (50) disposés à l'extérieur de la collerette cylindrique (46) pour évacuer vers l'atmosphère l'air expiré, caractérisé en ce que les moyens de retenue (30) soutiennent la membrane pendant l'expiration d'un malade afin d'empêcher un fléchissement de cette membrane (58) vers l'amont et comprennent un élément allongé (38) qui est disposé en regard de l'ouverture diamétrale (60) de la membrane et contre lequel cette membrane est appliquée au cours de l'expiration de façon à assurer l'étanchéité de ladite ouverture (60) en vue d'empêcher un écoulement de reflux lors de l'expiration, et en ce que la membrane est sensiblement plane, tandis que, lors d'une inspiration, la partie non perforée de la membrane qui fait face au rebord du siège de clapet se déplace jusqu'au contact de ce rebord de siège de clapet afin d'assurer l'étanchéité des moyens formant orifice d'évacuation (5) et que, lors d'une expiration, cette partie non perforée de la membrane quitte le contact de ce rebord du siège de clapet afin de libérer lesdits moyens formant orifice d'évacuation.

2. Clapet d'inhalation tel que revendiqué dans la revendication 1, caractérisé en ce que l'ouverture dimétrale est constituée par une fente (60).

3. Clapet d'inhalation tel que revendiqué dans la revendication 1 ou la revendication 2, caractérisé en ce que l'embout buccal comprend une plaquette transversale annulaire (44), une partie tubulaire (42) s'étendant en aval par rapport à cette plaquette transversale annulaire et au voisinage du bord intérieur de celle-ci afin de se loger dans la bouche d'un malade, ladite collerette annulaire (46) étant située à une certaine distance vers l'extérieur par rapport à cette partie tubulaire et s'étendant en amont de la plaquette transversale annulaire.

4. Clapet d'inhalation tel que revendiqué dans la revendication 3, caractérisé en ce que lesdits moyens formant orifice d'évacuation comprennent des ouvertures (50) ménagées dans la plaquette transversale annulaire (44) à l'extérieur de ladite collerette annulaire (46).

5. Clapet d'inhalation tel que revendiqué dans l'une quelconque des revendications 1 à 4, caractérisé en ce que les moyens de retenue sont constitués par un croisillon rayonnant (30) dont fait partie ladite pièce allongée (38).

6. Clapet d'inhalation tel que revendiqué dans la revendication 1, caractérisé en ce que les moyens récepteurs situés à l'extrémité d'entrée sont constitués par un élément tronconique à extrémité ouverte et en élastomère (20) qui est fixé sur l'extrémité d'entrée dudit corps et s'étend dans celui-ci.

FIG. 1

PRIOR ART

FIG. 2

FIG. 3

FIG. 2a

FIG. 4